# EUROPÄISCHE PATENTSCHRIFT

(11) **EP 2 198 898 B1**
(45) Veröffentlichungstag und Bekanntmachung des Hinweises auf die Patenterteilung: **10.01.2018**
(21) Anmeldenummer: 09177439.8
(22) Anmeldetag: 30.11.2009
(51) Int. Cl.: A61L 31/02, A61L 31/08, A61L 31/14

(54) **Implantat enthaltend Magnesium oder eine Magnesiumlegierung sowie Verfahren zur Herstellung einer Schichtstruktur**
Implant containing magnesium or a magnesium alloy and method for manufacturing
Implant contenant du magnesium ou un alliage de magnesium et methode de préparation d'une structure stratifiée

(30) Priorität: 18.12.2008 DE 102008054920
(43) Veröffentlichungstag der Anmeldung: 23.06.2010
(73) Patentinhaber: Biotronik VI Patent AG, 6341 Baar (CH)
(72) Erfinder: Bayer, Ullrich, 18211, Admannshagen-Bargeshagen (DE)
(74) Vertreter: Randoll, Sören

(56) Entgegenhaltungen:
- EP-A2- 2 172 234
- DE-A1-102006 060 501
- US-A1- 2004 254 604
- US-A1- 2007 237 946
- US-B1- 6 716 444
- HEUBLEIN B ET AL: "BIOCORROSION OF MAGNESIUM ALLOYS: A NEW PRINCIPLE IN CARDIOVASCULAR IMPLANT TECHNOLOGY?", HEART, BMJ, LONDON, GB, 1 January 2003 (2003-01-01), pages 651-656, XP002345264, ISSN: 1355-6037, DOI: 10.1136/HEART.89.6.651

## Beschreibung

Die Erfindung betrifft ein Implantat, insbesondere eine intraluminale Endoprothese, mit einem Körper, der Magnesium oder eine Magnesiumlegierung enthält, sowie ein Verfahren zur Herstellung einer Schichtstruktur auf einer Körperoberfläche eines derartigen Implantats.

Medizinische Endoprothesen oder Implantate für die unterschiedlichsten Anwendungen sind in großer Vielfalt aus dem Stand der Technik bekannt. Als Endoprothesen im Sinne der vorliegenden Erfindung sind endovaskuläre Prothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen ein rohrförmiges oder hohlzylinderförmiges Grundgitter auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderer Implantate kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für das Grundgitter biodegradierbarer Implantate geeignete Werkstoffe können beispielsweise Polymere oder Metalle enthalten. Das Grundgitter kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Magnesium oder eine Magnesiumlegierung.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Als für den genannten Zielkorridor der Degradation besonders vielversprechend haben sich biodegradierbare Implantate, die Magnesium oder eine Magnesiumlegierung enthalten, insbesondere sogenannte Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass sich bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemische Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende Degradation bzw. eine zu große Variabilität der Degradation des Implantats.

Ein weiteres Problem im Zusammenhang mit Passivierungsbeschichtungen ergibt sich daraus, dass Stents oder auch andere Implantate üblicherweise zwei Zustände annehmen können, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Auf Grund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus der Druckschrift DE 10 2006 060 501 ist ein Verfahren zur Herstellung einer korrosionshemmenden Beschichtung auf einem Implantat aus einer biokorrodierbaren Magnesiumlegierung sowie ein nach dem Verfahren erhältliches Implantat bekannt, bei dem nach Bereitstellen des Implantats die Implantatoberfläche mit einer wässrigen oder alkoholischen Konversionslösung enthaltend ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PO₃³⁻, PO₄³⁻, HPO₄²⁻, H₂PO₄⁻, OH⁻, BO₃³⁻, B₄O₇³⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻, wobei eine Konzentration des Ions oder der Ionen jeweils im Bereich von 10⁻² mol/l bis 2 mol/l liegt. Die Behandlung der Implantatoberfläche mit der genannten Konversionslösung bedingt eine anodische Oxidation des Implantats. Sie wird entweder ohne Verwendung einer äußeren Stromquelle (außen stromlos) oder mit einer Stromquelle durchgeführt. Die in dieser Druckschrift beschriebenen Verfahrensbeispiele sowie Elektrolytzusammensetzungen erfüllen jedoch die Erwartungen hinsichtlich Degradationsverhalten und Dilatationsfähigkeit ohne Schichtzerstörung bei der Anwendung für einen Magnesium-Stent nicht vollständig.

Die Aufgabe der vorliegenden Erfindung besteht somit darin, ein Verfahren zur Herstellung einer degradationshemmenden Schichtstruktur auf einem Körper eines Implantats anzugeben, welche eine Degradation des Implantats im gewünschten Zielkorridor ermöglicht. Dabei soll die Degradation zu einem kontrollierbaren Zeitpunkt stattfinden und zusätzlich eine Dilatation bzw. Verformung des Implantats ohne nennenswerten Einfluss auf das Degradationsverhalten ermöglichen. Entsprechend besteht die Aufgabe der Erfindung außerdem darin, ein derartiges Implantat zu schaffen.

Die obige Aufgabenstellung wird gelöst durch ein Implantat, das zumindest auf einem Teil seiner Körperoberfläche eine erste Schicht aufweist, die ein Wasserstoff bindendes Material, vorzugsweise Palladium, enthält. Hierbei wird unter einer Bindung von Wasserstoff sowohl das Einbinden von Wasserstoff in das Gitter des Wasserstoff bindenden Materials als auch die Bildung von Hydriden verstanden. Der Körper des Implantats umfasst mindestens einen Teil des Implantats, vorzugsweise den Hauptteil des Implantats, welcher die mechanische Integrität des Implantats bewirkt. Bei der Verwendung von Magnesium oder Magnesiumlegierungen für Implantate beim Kontakt mit körpereigenen Flüssigkeiten Degradationserscheinungen mit der Freisetzung von Wasserstoff als Reaktionsprodukt einhergehen (siehe B. Heublein, Heart 2003, 89, 651-653), Der Wasserstoff führt zu einer extremen lokalen Erniedrigung des pH-Wertes und einer damit verbundenen verstärkten Korrosion. Es wurde festgestellt, dass die Entstehung von Wasserstoff beim Kontakt von Magnesiumlegierungen mit wässrigen Medien nicht verhindert aber eine Verbesserung des Degradationsverhaltens dadurch erreicht werden kann, dass der Wasserstoff unmittelbar an seinem Entstehungsort, d. h. der Körperoberfläche des Implantats, stoffschlüssig oder formschlüssig über längere Zeiträume gebunden wird. Hierdurch kann sein Einfluss auf die Korrosionsgeschwindigkeit wesentlich reduziert werden.

Die klassische Herangehensweise bestand bisher darin, die Korrosionsgeschwindigkeit von Magnesiumimplantaten durch Aufbringen von verschiedenen Schutzschichten zu verlangsamen. Hierbei wurde das Hauptaugenmerk auf eine möglichst langzeitstabile, stoffliche Trennung zwischen dem Magnesiummaterial und dem Elektrolyten/Körperflüssigkeit gelegt. Beispiele dafür sind Konversionsschichten auf der Basis von Magnesiumfluorid, verschiedenartig aufgebrachte Depositionsschichten beispielsweise mit Titan, SiC, Magnesium, Magnesiumoxid oder anodisch erzeugten Mischverbindungen aus Magnesiumoxid und Magnesiumphosphat. Weitergehende Lösungsansätze gehen auch davon aus, dass das an der Körperoberfläche dominierende Reaktionsprodukt Kalziumphosphat vorher aufgebracht wird und somit die Reaktionsgeschwindigkeit des Korrosionsprozesses gebremst wird. Nachteilig an den bekannten Lösungen ist, dass diese nur eine geringe Versagenstoleranz aufweisen. Werden diese Schichten durch mechanische Einflüsse oder durch den Korrosionsangriff beschädigt und tritt in Folge dessen das Magnesiumgrundmaterial in direkten chemischen Kontakt mit dem Elektrolyten/Körperflüssigkeit, so kommt es zu einer lokal ungebremsten Korrosion. Die dabei entstehenden Wasserstoffionen diffundieren teilweise in die Oberfläche des Magnesiums ein und verspröden diese, ein anderer Teil rekombiniert zu molekularem Wasserstoff und bildet Blasen, die an der Magnesiumoberfläche adhärieren. Diese wachsen und werden durch Turbulenzen des Elektrolyten (Blut, Plasma usw.) von der Oberfläche abgerissen. Die Geometrie der Implantate bewirkt, dass die mit Pulsfrequenz vorbeiströmende Körperflüssigkeit unterschiedlich starke Turbulenzen und hierdurch lokal unterschiedliche Scherwirkungen auf die Wasserstoffblasen erzeugt. Dies hat zur Folge, dass jede dieser Blasen je nach Lage bezüglich des Implantats über eine unterschiedliche Zeitdauer auf die Implantatoberfläche einwirkt. Die damit einhergehenden Unterschiede in den Reaktionszeiten führen wiederum zu einem uneinheitlichen Korrosionsangriff und einer damit verbundenen unterschiedlichen Umwandlung von metallischem Magnesium zu Magnesiumhydroxid. Für den Fall, dass Korrosionsschutzschichten vorhanden sind (z. B. Kalziumphosphate, Magnesiumphosphate, Magnesiumfluorid oder Magnesiumoxid), besteht durch die Freisetzung von Wasserstoff bei der Degradation die Gefahr, dass die Schutzschichten durch den Wasserstoff unterwandert werden. Hierdurch bilden sich Fragmente des Körpers, die sich vom Implantat loslösen und ohne eine stoffliche Verbindung zu dem verbliebenen Restimplantat weiteren Korrosionsangriffen ausgesetzt sind. Das verbleibende Implantat unterliegt ebenfalls einer fortschreitenden Korrosion. Die hierdurch entstandene, größere Oberfläche zeigt Kavitäten, die den Korrosionsangriff weiter verstärken. Hierdurch entstehen zerklüftete Restquerschnitte und von Neointima umgebene korrodierte Bruchstücke. Letztendlich brechen die Stege des Implantats und es tritt ein Totalverlust der mechanischen Integrität des Magnesiumbauteils ein.

Der Verdienst des Erfinders ist es, in Abkehr von den bekannten Lösungen erkannt zu haben, dass durch eine Wasserstoff bindende, d.h. absorbierende, Beschichtung die beschriebenen schädlichen Wirkungen des Wasserstoffs, die durch die Freisetzung des Wasserstoffs als Reaktionsprodukt bei der Degradation verursacht werden, verhindert oder zumindest minimiert werden. Hierdurch kann die Degradation der degradierbaren Implantate wesentlich verlangsamt werden.

Die Wirksamkeit der in der ersten Schicht enthaltenen Palladiumteilchen als temporären Wasserstoffspeicher soll am Beispiel eines vaskulären Stents erläutert werden. Nanoskalige Palladiumteilchen, die, wie unten näher erläutert wird, bevorzugt verwendet werden, können in einer Umgebung mit Körperflüssigkeit das bis zu 12.000-fache Volumen an Wasserstoff speichern. Es ist ferner bekannt, dass bei einer Korrosion von 1 mg Magnesium in einem wässrigen Medium ca. 900 mm³ Wasserstoff freigesetzt wird. Eine vollständige Korrosion eines Magnesiumbauteils mit einer für vaskuläre Stents typischen Masse von 4 mg setzt also 3.600 mm³ Wasserstoff frei. Da eine ca. 5 µm dicke PalladiumSchicht auf einem Stent mit einer ca. 70 mm² großen Oberfläche ein Volumen von ca. 0,35 mm³ einnimmt, kann diese Schicht etwa 4.200 mm³ Wasserstoff binden (0,35 mm³ x 12.000). Eine Palladiumschicht mit einer Schichtdicke von 5 µm ist somit in der Lage, den gesamten frei werdenden Wasserstoff bei vollständiger Degradation des Stents zu binden. Selbst unter der Annahme, dass nicht das gesamte Palladium zu Palladiumhydrid umgewandelt wird, können durch eine 5 µm dicke Palladium enthaltende Schicht zumindest große Teile des bei der Degradation entstehenden Wasserstoffs chemisch gebunden werden. Das Palladium wirkt dabei im weitesten Sinne als "Opferelement". Durch den gebundenen Wasserstoff wird die korrosive Wirkung des das Implantat umgebenen Elektrolyten/der Körperflüssigkeit entschärft und hierdurch die Degradationsdauer des Magnesiumimplantats erhöht. Eine "normale" Degradation, d.h. eine Degradation unter Beteiligung von frei werdendem Wasserstoff, der nicht gebunden wird, setzt erst dann ein, wenn das gesamte Palladium, das in Kontakt mit dem Elektrolyten tritt, zu Palladiumhydrid umgewandelt ist. Das entstehende Palladiumhydrid verbleibt zunächst neben dem weiter korrodierenden Implantat im Gefäß, unterliegt einer fortschreitenden Fragmentierung bis in den Submikrometerbereich hinein und wird zum Teil auch von der umgebenden, körpereigenen Matrix inkorporiert.

Ein weiterer Vorteil einer Palladium-enthaltenden Beschichtung besteht darin, dass Palladium eine gute Röntgensichtbarkeit besitzt und daher zusätzlich als Röntgenmarker wirken kann. Selbst nach dem das Palladium in Palladiumhydrid umgewandelt wurde, zeigt dieses auf Grund seiner hohen Dichte von ca. 12 g/cm³ und seiner Ordnungszahl 46 immer noch eine gute Röntgensichtbarkeit und kann somit auch nach der Umwandlung, wenn es neben dem Implantat im Gefäß verbleibt, mittels eines Röntgengeräts erkannt werden und somit insbesondere bei Verwendung einer hohen Röntgenenergiedichte auch als Indikator für die Degradation des Magnesiumbauteils herangezogen werden.

In einem bevorzugten Ausführungsbeispiel beträgt die Konzentration des Wasserstoff bindenden Materials, insbesondere des Palladiums, in der ersten Schicht zwischen 90 Gew.% und 100 Gew.%. Bei einer derartigen Konzentration kann besonders viel Wasserstoff gebunden werden.

Die erste Schicht kann beispielsweise eine Schichtdicke von 1 µm bis 10 µm, vorzugsweise 2 µm bis 6 µm aufweisen. Wie bereits oben anhand eines Beispiels dargelegt wurde, kann mit einem Schichtvolumen beruhend auf eine Schichtdicke von wenigen Mikrometern eine ausreichende Menge von Wasserstoff gebunden werden, so dass die Degradation des degradierbaren Implantats wesentlich verlangsamt wird.

In einem weiteren Ausführungsbeispiel liegen die mittleren Korngrößen der Palladiumteilchen zwischen 20 nm und 15 µm, vorzugsweise zwischen 50 nm und 500 nm. Die mittlere Korngröße wurde hierbei mittels rasterelektronenmikroskopischer Verfahren als arithmetisches Mittel der vermessenen Körner bestimmt. Insbesondere nanoskalige Palladiumteilchen können ein Vielfaches ihres Volumens an Wasserstoff speichern. Demzufolge ist es von Vorteil, möglichst Korngrößen in dem angegebenen Bereich zu wählen.

In einem weiteren Ausführungsbeispiel weist das Implantat zumindest auf einem Teil seiner Körperoberfläche eine über der ersten Schicht angeordnete zweite Schicht, die vorzugsweise Parylene und/oder Magnesiumstearat enthält, auf.

Durch eine Parylene-Schutzschicht kann die Degradationszeit des Implantats nochmals wesentlich erhöht werden, wobei bevorzugte Schichtdicken der Parylene-Schicht zwischen 0,5 µm und 10 µm liegen. Hierbei ist Parylene die Bezeichnung für vollständig lineare, teilkristalline und unvernetzte aromatische Polymere. Die verschiedenen Polymere besitzen unterschiedliche Eigenschaften und lassen sich in vier Grundtypen einteilen, nämlich Parylene C, Parylene D, Parylene N und Parylene F.

Auch eine magnesiumstearathaltige Beschichtung wirkt sich vorteilhaft auf die Eigenschaften des Implantats aus. Die Magnesiumstearat-Beschichtung bewirkt eine höhere Adhäsion der Palladiumartikel an der Körperoberfläche oder an einer darunter liegenden Zwischenschicht, insbesondere wenn diese Poren aufweist. Die Magnesiumstearat-Beschichtung weist vorzugsweise eine Schichtdicke auf, die zwischen 0,5 µm und 10 µm liegt.

In einem weiteren Ausführungsbeispiel ist zwischen der Körperoberfläche und der ersten Schicht eine vorzugsweise mittels plasmachemischer Behandlung erzeugte Zwischenschicht angeordnet, die zumindest auf einem Teil der Körperoberfläche des Implantats vorliegt und porös ist, wobei die Dicke der Zwischenschicht vorzugsweise 1 µm bis 20 µm, besonders bevorzugt 2 µm bis 8 µm beträgt. Die Zwischenschicht weist in einem besonders bevorzugten Ausführungsbeispiel mindestens eine Verbindung ausgewählt aus der Gruppe enthaltend Phosphate, Hydroxide und Oxide des biodegradierbaren metallischen Materials oder der biodegradierbaren metallischen Materialen des Implantatkörpers auf.

Die plasmachemische Behandlung umfasst dabei die Behandlung in einem wässrigen Elektrolytsystem (wässrige Lösung), bei der plasmachemische Effekte direkt an der Oberfläche des Körpers des Implantats entstehen. Das für Mikrosekunden stabile Plasma an der Körperoberfläche erzeugt Reaktionsprodukte, welche zur Ausbildung der Zwischenschicht auf der Körperoberfläche führen. Die Zwischenschicht wird vorzugsweise derart auf einem entsprechenden Teil der oder der gesamten Körperoberfläche des Implantats angeordnet, dass die Zwischenschicht zwischen der Körperoberfläche und der ersten Schicht, welche ein Wasserstoff bindendes Material enthält, liegt.

Durch die plasmachemische Behandlung bilden sich auf der Körperoberfläche des Implantats Phosphate, Hydroxide und Oxide des metallischen Materials, insbesondere des Magnesiums. Diese Schichtzusammensetzung stellt bei Kontakt mit der Körperflüssigkeit einen temporären Korrosionsschutz dar, der eine verzögerte Degradation des metallischen Materials bewirkt. Die im Zuge der verzögerten Degradation des Implantats frei werdenden Partikel werden teilweise von körpereigenen Zellen inkorporiert und/oder weiter abgebaut.

Ein weiterer Vorteil einer mittels einem plasmachemischen Prozess erzeugten Schicht besteht darin, dass die durch die vorgeschalteten Oberflächenbehandlungsprozesse des Implantats nicht entfernbaren Oberflächenkontaminationen des Grundmaterials durch die degradationshemmende Zwischenschicht absorbiert werden und somit den Degradationsprozess nicht noch zusätzlich negativ beeinflussen. Zudem werden aus der Oberfläche herausragende, teilweise scharfkantige Ausscheidungen des Implantat-Körpers (wie zum Beispiel nicht gelöste Legierungsbestandteile, z. B. Yttrium und dessen Verbindungen) oder Rückstände von der davor erfolgten Be- und Verarbeitung des Implantatkörpers abgedeckt. Daraus ergibt sich eine noch weiter erhöhte Hämo- bzw. Biokompatibilität. Außerdem weist die verfahrensbedingt poröse Struktur der Zwischenschicht ein hohes plastisches Verformungsvermögen auf. Beispielsweise werden die beim Dilatieren eines Stents entstehenden Mikrorisse durch Energieakkumulation beziehungsweise -dissipation in den zu den Mikrorissen benachbarten Poren gestoppt. Es kommt somit nicht zu einer Delamination der Zwischenschicht.

Die obige Aufgabe wird ferner durch ein Verfahren zur Herstellung einer degradationshemmenden Schichtstruktur (oder einer degradationshemmenden Beschichtung) auf einer Körperoberfläche eines Implantats, wobei der Körper mindestens Magnesium oder eine Magnesiumlegierung enthält, gelöst, das die folgenden Schritte umfasst:
a) Bereitstellen des Körpers des Implantats,
b) Aufbringen der ersten Schicht auf mindestens einen Teil der Körperoberfläche, wobei die erste Schicht ein Wasserstoff bindendes Material, vorzugsweise Palladium enthält.

Die Anwendung des erfindungsgemäßen Beschichtungsverfahrens zur Herstellung einer ersten Schicht mit einem Wasserstoff bindenden Material ist einfach und kostengünstig, um ein erfindungsgemäßes Implantat mit den oben beschriebenen Vorteilen herzustellen. In einem bevorzugten Ausführungsbeispiel wird die erste Schicht mittels Eintauchen des Körpers des Implantats in eine Lösung aufgebracht, die vorzugsweise Palladium und ein Elektrolyt enthält, wobei der Elektrolyt beispielsweise tensidhaltiges wässriges oder kohlenwasserstoffhaltiges Elektrolyt ist. Die Anwendung eines Tauchprozesses zum Aufbringen der ersten Schicht ist besonders kostengünstig, da dieser wenig Energie verbraucht.

Eine Beschichtung mit der gewünschten, oben angegeben Schichtdicke wird in vorteilhafter Weise erreicht, wenn der Körper des Implantats über einen Zeitraum von 10 Sekunden bis 30 Sekunden in der Lösung verbleibt.

Alternativ zur Verwendung eines Tauchverfahrens kann die erste Schicht auch mittels einer galvanischer Behandlung in einer wässrigen Lösung aufgebracht werden, bei der eine elektrische Spannung an den Körper des Implantats angelegt wird, wobei die wässrige Lösung vorzugsweise ein wasserlösliches Leitsalz, beispielsweise Kaliumdihydrogenphosphat, einen Komplexbildner, beispielsweise Zitronensäure, Ethylendiamintetraessigsäure und/oder Weinsäure, sowie eine palladiumhaltige Metallverbindung, beispielsweise eine Verbindung aus der Gruppe enthaltend Palladium(II)Acetat-Trimer und Tris(dibenzylidenaceton)dipalladium(0), enthält.

Insbesondere nach dem Auftragen einer plasmachemisch erzeugten Zwischenschicht ist das Aufbringen einer ersten Schicht mittels galvanischer Behandlung von Vorteil, da nach dem Auftragen der Zwischenschicht lediglich ein oder mehrere Spülvorgänge in destilliertem Wasser durchgeführt werden müssen. Die Beschichtung im palladiumhaltigen Elektrolyt erfolgt anschließend nach dem kathodischen Verfahrensprinzip. Das bedeutet, dass das bereits plasmachemisch beschichtete Mg-Substrat als Kathode geschaltet wird. Als Anode dient z.B. ein umlaufendes platiniertes Titanblech. Die Beschichtung erfolgt im Niederspannungsbereich unter Verwendung von Gleichspannung oder gepulster Gleichspannung von maximal 40 V.

Bei diesem Verfahren ist außerdem von Vorteil, dass sich während des Aufbringens der ersten Schicht die positiv geladenen Palladiumionen in den Zonen größter elektrischer Felddichte des Magnesiumbauteils anlagern. Diese Zonen sind bei Vorhandensein einer plasmachemisch aufgetragenen Zwischenschicht die in diesem Beschichtungsprozess entstandenen Poren. Die Palladiumionen werden am Porengrund und an den Porenwänden zu metallischem Palladium reduziert. Ein direkter metallischer Kontakt zum Material des Implantatkörpers mit Magnesium oder einer Magnesiumlegierung findet nicht statt, da der Porengrund eine bis zu 1 µm dicke Barriereschicht aus einer Mischung von Oxiden, Hydroxiden und Phosphaten aufweist. In einem weiteren bevorzugten Ausführungsbeispiel kann nach einem anschließenden Spülen in einem geeigneten Lösungsmittel ein abschließender Tauchvorgang in einem stark alkalischen Medium (beispielsweise 25%-ige Ammoniaklösung oder 2 bis 4 molare Natronlauge) durchgeführt werden, der zu einer Verengung der Porenhälse und damit einer verbesserten Verankerung des Palladiums auf dem Porengrund und an den Porenwänden führt.

Wie bereits erläutert, wird vor dem Aufbringen der ersten Schicht bevorzugt mindestens ein Teil der Körperoberfläche einer plasmachemischen Behandlung in einer wässrigen Lösung zur Erzeugung einer Zwischenschicht unterzogen, bei der eine das Plasma erzeugende elektrische Spannung an den Körper des Implantats angelegt wird. Hierbei weist die wässrige Lösung zur Erzeugung der Zwischenschicht vorzugsweise eine lonenart oder mehrere lonenarten ausgewählt aus der Gruppe der Phosphationen, Kaliumionen und Kalziumionen auf.

Bevorzugt enthält die Zwischenschicht mindestens eine Verbindung ausgewählt aus der Gruppe Phosphate, Hydroxide und Oxide des biodegradierbaren Materials.

Oben wurde bereits beschrieben, dass die Zwischenschicht Poren aufweist, wobei vorzugsweise auf dem Porengrund und an den Seitenwänden der Poren eine erste Schicht mit Wasserstoff bindendem Material, vorzugsweise Palladium, gebildet ist, nachdem die erste Schicht, vorzugsweise mittels galvanischer Behandlung, aufgebracht ist.

Um einen oben beschriebenen vorteilhaften Schutz der ersten Schicht zu erreichen, wird in einem Ausführungsbeispiel auf die erste Schicht eine zweite Schicht, die vorzugsweise Parylene und/oder Magnesiumstearat enthält, aufgebracht.

Die obige Aufgabenstellung wird ferner gelöst durch ein Implantat erhältlich durch das oben beschriebene erfindungsgemäße Verfahren. Diese erfindungsgemäßen Implantate weisen die oben im Zusammenhang mit dem erfindungsgemäßen Herstellungsverfahren angegebenen Vorteile auf.

Das erfindungsgemäße Verfahren wird nachfolgend anhand von Beispielen erläutert. Dabei bilden alle beschriebenen Merkmale den Gegenstand der Erfindung, unabhängig von ihrer Zusammenfassung in den Ansprüchen oder deren Rückbeziehung.

### 1. Beispiel: Herstellung eines Implantats mit plasmachemisch erzeugter Phosphatschicht mit kathodischer Nachbehandlung in palladiumhaltigem Elektrolyt

Der Körper eines Implantats, vorzugsweise eines endovaskulären Stents, wird zunächst mit Hilfe bekannter Technologien wie Laserschneiden, anschließendem Entgraten und Elektropolieren hergestellt.

Danach erfolgt ein anodisches Kontaktieren in einem Kalium-, Calcium- und Phosphorionen enthaltenden wässrigen Elektrolyt folgender Zusammensetzung zur plasmachemischen Beschichtung:
- 65 ml/l Ethylendiamin-Lösung (99%),
- 80 g/l Kaliumdihydrogenphosphat und/ oder jeweils 50 g/l Calciumcarbonat,
- oder Calciumcitrat oder Calcium-Citrat-Malat,
- 20 ml/l wässrige Ammoniumhydroxidlösung (25%)
- 25 g/l Natriumcarbonat.

Für die plasmachemische Beschichtung werden gepulste Spannungen zwischen 250 V und 500 V (ständig steigende Spannung), Stromdichten zwischen 0,5 und 5 A/dm² sowie Pulsfrequenzen zwischen 100 Hz und 10 kHz gewählt. Die Gegenkatode besteht aus Edelstahl 1.4301. Die unter stetig steigender Badspannung einsetzende plasmachemische Reaktion führt zu verfahrensbedingt porösen Oberflächen. Die Poren weisen Durchmesser zwischen 0,1 und 8 µm auf. Als Reaktionsprodukt der plasmachemischen Reaktion entsteht eine Oxide, Hydroxide sowie Phosphate enthaltende Oberflächen- und Zwischenschicht, die außerdem Elemente und Verbindungen des Materials des Implantat-Körpers enthält. Abschließend erfolgt ein zweistufiges Spülen in einem geeigneten Lösungsmittel.

Unmittelbar danach wird der immer noch mit einem Titan- oder Aluminiumdraht kontaktierte Stent in eine andere erfindungsgemäße Elektrolytlösung definierter elektrischer Leitfähigkeit getaucht. Diese enthält neben einem wasserlöslichen Leitsalz (z.B. Kaliumdihydrogenphosphat) und Komplexbildnern wie z.B. Zitronensäure, Ethylendiamintetraessigsäure, Weinsäure auch noch Metallverbindungen wie Palladium(II)Acetat-Trimer [Pd(O₂CCH₃)₂]₃ oder Tris(dibenzylidenaceton)dipalladium(0) [(C₁₇H₁₄O)Pd₂]₃. Vor dem nachfolgenden Beschichtungsprozess wird das Magnesiumbauteil nunmehr kathodisch geschaltet und mit einer konstanten oder gepulsten Badspannung beaufschlagt, die maximal 40 V beträgt. Während des galvanischen Prozesses lagern sich Palladiumionen in den Zonen größter elektrischer Felddichte des Magnesiumbauteils an. Dies sind die aus dem vorangegangenen Beschichtungsprozess entstandenen Poren. Die Palladiumionen werden am Porengrund und an den Porenwänden zu metallischem Palladium reduziert. Ein direkter metallischer Kontakt zum Magnesiumgrundmaterial findet dabei nicht statt, da der Porengrund eine bis zu 1 µm dicke Barriereschicht aus Oxiden, Hydroxiden und Phosphaten aufweist. Nach der Beendigung des Prozesses erfolgt ein mehrmaliges Spülen in geeigneten Lösungsmitteln. Ein abschließender Tauchvorgang in einem stark alkalischen Medium (z.B. wässrige 25%-ige Ammoniaklösung oder 2 bis 4 molare Natronlauge) führt zu einer Verengung der Porenhälse und damit zu einer besseren Verankerung des Palladiums auf dem Porengrund und an den Porenwänden.

### 2. Beispiel: Herstellung eines Implantats mit plasmachemisch erzeugter Phosphatschicht mit stromloser Nachbehandlung in kolloidalem palladiumhaltigen Elektrolyt

Zunächst wird der Implantat-Körper wie in Beispiel 1 beschrieben hergestellt und eine plasmachemische Beschichtung des Implantat-Körpers gemäß Beispiel 1 durchgeführt, die mit dem zweistufigen Spülen abgeschlossen wird. Anschließend erfolgt statt der in Beispiel 1 erläuterten galvanischen Beschichtung eine stromlose Nachbehandlung in einer kolloidalen Palladiumlösung (30 mg/l bis 5 g/l Pd) über einen Zeitraum von etwa 0,5 Minuten bis etwa 10 Minuten, wobei die Lösung als Kolloidstabilisator auch Zinn (II) Salze enthalten kann.

### 3. Beispiel: Herstellung eines Implantats mit plasmachemisch erzeugter Phosphatschicht mit stromloser Nachbehandlung in kolloidalem palladiumhaltigen Elektrolyt mit finaler Behandlung (Tauchen in Magnesiumstearat)

Zunächst wird das Verfahren gemäß Beispiel 2 (einschließlich Vorgehen gemäß Beispiel 1) durchgeführt. Danach wird der Implantat-Körper auf einen Kunststofffaden (z.B. Polyamid) aufgehängt und anschließend in eine Lösung zur Auftragung des Magnesiumstearats getaucht. Die Lösung besteht z.B. aus 9 Teilen hochreinem Aceton oder Isopropanol oder Tetrahydrofuran und 1 Teil Magnesiumstearat (Teile jeweils bezogen auf das Gewicht der jeweiligen Komponenten). Der Tauchvorgang erfolgt bei Temperaturen zwischen Raumtemperatur und dem jeweiligen Siedepunkt des Lösungsmittels (z.B. 56°C bei Aceton, 82°C bei Isopropanol und 64°C bei Tetrahydrufuran. Diese Temperaturen erniedrigen sich, wenn der Tauchvorgang in einem evakuierbaren Exsikkator durchgeführt wird. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Implantatoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt im Bereich von etwa 0,5 bis etwa 10 µm.

Bedingt durch den im Exsikkator vorliegenden Unterdruck wird das Magnesiumstearat sehr gleichmäßig auf der Oberfläche des Implantat-Körpers abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht erhalten wird. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

### 4. Beispiel: Herstellung eines Implantats mittels Oberflächenbehandlungen gemäß Beispiele 2 oder 3 ohne vorangegangene plasmachemische Behandlung

Die obigen, anhand der Beispiele 2 oder 3 erläuterten Verfahren können auch ohne eine vorausgegangene plasmachemische Behandlung ausgehend von dem mittels Laserschneiden, anschließendem Entgraten und Elektropolieren hergestellten Implantat-Körper durchgeführt werden.

### 5. Beispiel: Herstellung eines Implantats mittels eines Tauchprozesses

Eine weitere Möglichkeit der Oberflächenbeladung mit Palladium besteht in der Anwendung eines Tauchprozesses. Hierbei wird in einen tensidhaltigen wässrigen oder kohlenwasserstoffenthaltenden Elektrolyt nanoskaliges (bzw. mikroskaliges) Palladium eingerührt. Der Partikeldurchmesser kann dabei zwischen 20 Nanometern und 15 Mikrometern variieren. Der Tensidanteil trägt dazu bei, dass die Grenzflächenspannung des nanoskaligen Palladiums herabgesetzt wird. Die sich bildende Emulsion hält die Palladiumteilchen in der Schwebe. Die vorher in der Elektrolytlösung plasmachemisch beschichteten Magnesium-Implantat-Körper (siehe Beispiel 1) werden stromlos in das je nach Konzentration des Palladiumpulvers leicht trübe bis undurchsichtige Gemisch getaucht. Dabei wird die Lösung mittels Magnetrührer so bewegt, dass sich keine Konzentrationsunterschiede an Palladiumteilchen im Beschichtungsbehälter ergeben. Der Stent verbleibt zwischen 30 Sekunden und 10 Minuten in der Lösung, wird anschließend herausgezogen und sofort in einen Trocknungsofen verbracht. Nach dem Antrocknen verbleibt eine mehrere Mikrometer dicke, nanoskalige Palladiumteilchen enthaltende Schicht auf dem Stent.

Um ein Abbröckeln dieser Schicht bei nachfolgenden Montageprozessen zu verhindern, erfährt das Magnesiumimplantat eine finale Tauchbehandlung in einer Magnesiumstearat enthalten Lösung zur Herstellung der zweiten Schicht. Danach wird das Bauteil entweder verpackt und/oder -im Falle von Stents - auf den Katheter montiert.

Alternativ kann auch ein unbeschichteter (nur elektropolierter) Magnesiumimplantat-Körper in die oben angegebene Palladiumlösung getaucht werden.

### 6. Beispiel: Aufbringen der Parylene enthaltenden zweiten Schicht

Zunächst wird das Implantat analog zu einem der in den Beispielen 1 bis 5 angegebenen Verfahren hergestellt. Anschließend wird (in Beispiel 5 anstelle der Magnesiumstearatbeschichtung) mittels eines CVD- Prozesses eine etwa 0,5 µm bis etwa 7 µm dicke Parylene C Schicht oder Parylene N Schicht aufgetragen.

Die zu behandelnden Stents werden dabei in einer Beschichtungskammer platziert. Mit einer auf das Kammervolumen und die Stentoberfläche berücksichtigenden Einwaage (z.B. ca. 4 g bei 1 µm und ca. 10 g bei 3 µm angestrebter Schichtdicke) wird der Beschichtungsprozess gestartet. Die Verdampfertemperatur beträgt dabei zwischen 100°C und 170°C. Das pulverförmige Monomer wird auf Grund des angelegten Kammervakuums von ca. 0,5 Pa bis 50 Pa über eine Heizplatte gesaugt. Die Temperatur der Heizplatte beträgt dabei zwischen 650°C und 730°C. Nach einer Beschichtungszeit von ca. 1 Stunde (bei einer angestrebten Schichtdicke von 1 µm) oder 3 Stunden (bei einer angestrebten Schichtdicke von 3 µm) weisen die Implantate eine homogene Bedeckung mit Parylene C (bzw. N) auf.

### 7. Beispiel: Herstellung eines porösen Implantats mittels eines Tauchverfahren

Ein geschäumter offenporiger Magnesiumimplantat-Körper wird bei Unterdruck in eine Palladiumteilchen enthaltende Lösung getaucht. Es findet eine Infiltration der Poren mit dieser Suspension statt. Ein nachfolgendes Tempern bei Temperaturen zwischen etwa 100°C und etwa 250°C führt zu einer Verdampfung des Lösungsmittels. Die Palladiumpartikel verbleiben in der Porenstruktur.

Es kann sich eine nachfolgende Versiegelung in einer Magnesiumstearat-haltigen Lösung bei Unterdruck anschließen. Diese bewirkt eine höhere Adhäsion der Palladiumpartikel in der Porenstruktur. Eine finale Warmauslagerung bei Temperaturen zwischen 80°C und 150°C führt zu einem weitestgehenden Porenverschluss und reduziert die mit dem Körper in Kontakt tretende reale Bauteiloberfläche.

In einem weiteren bevorzugten Ausführungsbeispiel wird der Implantat-Körper vor der plasmachemischen Behandlung zur Herstellung der Zwischenschicht oder der ersten Schicht elektrochemisch behandelt, vorzugsweise elektrochemisch poliert. Hierdurch werden Verunreinigungen auf der Oberfläche des Implantat-Körpers entfernt, so dass die plasmachemische Behandlung an einer definierten Oberfläche erfolgt.

Bevorzugt erfolgt die plasmachemische Behandlung des Implantat-Körpers zur Erzeugung der Zwischenschicht dadurch, dass an den Körper eine gepulste, positive, Spannung angelegt wird, deren Amplitude im überwiegenden Zeitraum der Behandlung mindestens etwa 90 Volt übersteigt, besonders bevorzugt mindestens etwa 100 Volt übersteigt und welche vorzugsweise im Verlauf der Behandlung ansteigt. Durch diese hohen gepulsten Spannungen mit einer Pulslänge von vorzugsweise maximal etwa 20 Mikrosekunden, besonders bevorzugt etwa 5 Mikrosekunden, werden an der Oberfläche des Implantat-Körpers über Mikrosekunden Plasmen erzeugt, welche zur Reaktion des metallischen Materials des Implantatkörpers mit dem Elektrolyten führen. Zwischen den Spannungspulsen folgt eine Ruhephase von vorzugsweise ca. 100 Mikrosekunden. Bevorzugt wird der plasmachemische Prozess mit einer Stromdichte von mindestens etwa 5 mA/cm², vorzugsweise mindestens etwa 10 mA/cm², durchgeführt.

Mittels des beschriebenen Verfahrens hergestellte Implantate degradieren in dem gewünschten Zeitfenster. Überraschender Weise lässt sich hierbei die Degradation u.a. durch eine Schicht (Zwischenschicht) steuern, welche porös ausgebildet ist.

## Patentansprüche

1. Implantat, insbesondere intraluminale Endoprothese, mit einem Körper, der mindestens Magnesium oder eine Magnesiumlegierung enthält, **dadurch gekennzeichnet, dass** das Implantat zumindest auf einem Teil seiner Körperoberfläche eine erste Schicht aufweist, die ein Wasserstoff bindendes Material, vorzugsweise Palladium, enthält.

2. Implantat nach Anspruch 1, **dadurch gekennzeichnet, dass** die erste Schicht eine Schichtdicke von 1 µm bis 10 µm, vorzugsweise **2** µm bis 6 µm aufweist.

3. Implantat nach einem der vorhergehenden Ansprüche, **dadurch gekennzeichnet, dass** die Korngrößen der Palladiumteilchen zwischen 20 nm und 15 µm, vorzugsweise zwischen 50 nm bis 500 nm, liegen.

4. Implantat nach einem der vorhergehenden Ansprüche, **dadurch gekennzeichnet, dass** das Implantat zumindest auf einem Teil seiner Körperoberfläche eine über der ersten Schicht angeordnete zweite Schicht, die vorzugsweise Parylene und/oder Magnesiumstearat enthält, aufweist.

5. Implantat nach Anspruch 4, **dadurch gekennzeichnet, dass** die zweite Schicht eine Schichtdicke von 0,5 µm bis 10 µm aufweist.

6. Implantat nach einem der Ansprüche 1 bis 5, **dadurch gekennzeichnet, dass** zwischen der Körperoberfläche und der ersten Schicht eine vorzugsweise mittels plasmachemischer Behandlung erzeugte Zwischenschicht angeordnet ist, die zumindest auf einem Teil der Körperoberfläche des Implantats vorliegt und porös ist, wobei die Dicke der Zwischenschicht vorzugsweise etwa 1 µm bis etwa 20 µm, besonders bevorzugt etwa 2 µm bis etwa 8 µm beträgt.

7. Implantat nach Anspruch 6, **dadurch gekennzeichnet, dass** die Zwischenschicht mindestens eine Verbindung ausgewählt aus der Gruppe enthaltend Phosphate, Hydroxide und Oxide des biodegradierbaren metallischen Materials oder der biodegradierbaren metallischen Materialien des Implantatkörpers aufweist.

8. Verfahren zur Herstellung einer degradationshemmenden Schichtstruktur auf einer Körperoberfläche eines Implantats, insbesondere einer intraluminalen Endoprothese, wobei der Körper mindestens Magnesium oder eine Magnesiumlegierung enthält, umfassend die folgenden Schritte:
a) Bereitstellen des Körpers des Implantats,
b) Aufbringen einer ersten Schicht auf mindestens einen Teil der Körperoberfläche, wobei die erste Schicht ein Wasserstoff bindendes Material, vorzugsweise Palladium, enthält.

9. Verfahren nach Anspruch 8, **dadurch gekennzeichnet, dass** die erste Schicht mittels Eintauchen des Implantatkörpers in eine Lösung aufgebracht wird, die vorzugsweise Palladium und ein Elektrolyt, beispielsweise tensidhaltiges wässriges oder kohlenwasserstoffhaltiges Elektrolyt, enthält.

10. Verfahren nach Anspruch 9, **dadurch gekennzeichnet, dass** der Körper des Implantats über einen Zeitraum von etwa 10 Sekunden bis etwa 30 Sekunden in der Lösung verbleibt.

11. Verfahren nach Anspruch 8, **dadurch gekennzeichnet, dass** die erste Schicht mittels einer galvanischen Behandlung in einer wässrigen Lösung aufgebracht wird, wobei die wässrige Lösung vorzugsweise ein wasserlösliches Leitsalz, beispielsweise Kaliumdihydrogenphosphat, einen Komplexbildner, beispielsweise Zitronensäure, Ethylendiamintetraessigsäure und/oder Weinsäure, sowie eine palladiumhaltige Metallverbindung, beispielsweise eine Verbindung aus der Gruppe enthaltend Palladium(II)Acetat-Trimer und Tris(dibenzylidenaceton)dipalladium(0), enthält.

12. Verfahren nach einem der Ansprüche 8 bis 11, **dadurch gekennzeichnet, dass** vor dem Aufbringen der ersten Schicht mindestens ein Teil der Körperoberfläche einer plasmachemischen Behandlung in einer wässrigen Lösung zur Erzeugung einer Zwischenschicht unterzogen wird, bei der eine das Plasma erzeugende elektrische Spannung an den Körper des Implantats angelegt wird.

13. Verfahren nach Anspruch 12, **dadurch gekennzeichnet, dass** die wässrige Lösung zur Erzeugung der Zwischenschicht ein oder mehrere Ionen ausgewählt aus der Gruppe der Phosphationen, Kaliumionen und Calciumionen aufweist.

14. Verfahren nach einem der Ansprüche, 8-13
**dadurch gekennzeichnet,**
**dass** auf die erste Schicht eine zweite Schicht, die vorzugsweise Parylene und/oder Magnesiumstearat enthält, aufgebracht wird.

## Claims

1. An implant, in particular an intraluminal endoprosthesis, having a body comprising at least magnesium or a magnesium alloy, **characterised in that** the implant has a first layer containing a hydrogen-binding material, preferably palladium, on at least a portion of its body surface.

2. The implant according to claim 1, **characterised in that** the first layer has a layer thickness of 1 µm to 10 µm, preferably 2 µm to 6 µm.

3. The implant according to either one of the preceding claims, **characterised in that** the grain sizes of the palladium particles are between 20 nm and 15 µm, preferably between 50 nm and 500 nm.

4. The implant according to any one of the preceding claims, **characterised in that** the implant has a second layer, preferably containing parylene and/or magnesium stearate, arranged over the first layer on at least a portion of the body surface of the implant.

5. The implant according to claim 4, **characterised in that** the second layer has a layer thickness of 0.5 µm to 10 µm.

6. The implant according to any one of claims 1 to 5, **characterised in that** an intermediate layer, preferably created by a plasma chemical treatment, is arranged between the body surface and the first layer, said intermediate layer being porous and being provided over at least a portion of the body surface of the implant, wherein the thickness of the intermediate layer is approximately 1 µm to approximately 20 µm, particularly preferably approximately 2 µm to approximately 8 µm.

7. The implant according to claim 6, **characterised in that** the intermediate layer comprises at least one compound selected from the group containing phosphates, hydroxides and oxides of the biodegradable metallic material or the biodegradable metallic materials of the implant body.

8. A method for producing a degradation-inhibiting layer structure on a body surface of an implant, in particular an intraluminal endoprosthesis, wherein the body contains at least magnesium or a magnesium alloy, said method comprising the following steps:
a) preparing the body of the implant, and
b) applying a first layer to at least part of the body surface, wherein the first layer contains a hydrogen-binding material, preferably palladium.

9. The method according to claim 8, **characterised in that** the first layer is applied by immersing the implant body in a solution, preferably containing palladium and an electrolyte, for example an aqueous electrolyte containing a surfactant or an electrolyte containing a hydrocarbon.

10. The method according to claim 9, **characterised in that** the body of the implant remains in the solution for a period of approximately 10 seconds to approximately 30 seconds.

11. The method according to claim 8, **characterised in that** the first layer is applied by a galvanic treatment in an aqueous solution, wherein the aqueous solution preferably contains a water-soluble conductive salt, for example potassium dihydrogen phosphate, a chelating agent, for example citric acid, ethylenediaminetetraacetic acid and/or tartaric acid, as well as a metal compound containing palladium, for example a compound from the group containing palladium(II) acetate trimer and tris(dibenzylideneacetone)dipalladium(0).

12. The method according to any one of claims 8 to 11, **characterised in that,** before applying the first layer, at least part of the body surface is subjected to a plasma chemical treatment in an aqueous solution to produce an intermediate layer, during which treatment an electric voltage generating the plasma is applied to the body of the implant.

13. The method according to claim 12, **characterised in that** the aqueous solution for producing the intermediate layer contains one or more ions, selected from the group of phosphate ions, potassium ions and calcium ions.

14. The method according to any one of claims 8 to 13, **characterised in that** a second layer, preferably containing parylene and/or magnesium stearate, is applied to the first layer.

## Revendications

1. Implant, notamment endoprothèse intraluminale, avec un corps qui contient au moins du magnésium ou un alliage de magnésium, **caractérisé en ce que** l'implant présente au moins sur une partie de sa surface corporelle une première couche qui contient un matériau liant l'hydrogène, de préférence du palladium.

2. Implant selon la revendication 1, **caractérisé en ce que** la première couche présente une épaisseur de couche de 1 µm à 10 µm, de préférence de 2 µm à 6 µm.

3. Implant selon l'une des revendications précédentes, **caractérisé en ce que** les tailles granulaires des particules de palladium se situent entre 20 nm et 15 µm, de préférence entre 50 nm et 500 nm.

4. Implant selon l'une des revendications précédentes, **caractérisé en ce que** l'implant présente au moins sur une partie de sa surface corporelle une deuxième couche disposée au dessus de la première couche qui contient de préférence du parylène et/ou du stéarate de magnésium.

5. Implant selon la revendication 4, **caractérisé en ce que** la deuxième couche présente une épaisseur de couche de 0,5 µm à 10 µm.

6. Implant selon l'une des revendications 1 à 5, **caractérisé en ce q**u'entre la surface corporelle et la première couche, il y a une couche intermédiaire générée de préférence au moyen d'un traitement chimique par plasma qui est présente sur au moins une partie de la surface corporelle de l'implant, où l'épaisseur de la couche intermédiaire s'élève de préférence d'environ 1 µm à environ 20 µM, de manière particulièrement préférée d'environ 2 µm à environ 8 µm.

7. Implant selon la revendication 6, **caractérisé en ce que** la couche intermédiaire présente un composé choisi dans le groupe contenant des phosphates, des hydroxydes et des oxydes du matériau métallique biodégradable ou des matériaux métalliques biodégradables du corps d'implant.

8. Procédé de fabrication d'une structure en couches inhibitrice de dégradation sur une surface corporelle d'un implant, notamment d'une endoprothèse intraluminale, où le corps contient au moins du magnésium ou un alliage de magnésium, comprenant les étapes suivantes :
a) fourniture du corps de l'implant,
b) dépôt d'une première couche sur au moins une partie de la surface corporelle, où la première couche contient un matériau fixant l'hydrogène, de préférence, du palladium.

9. Procédé selon la revendication 8, **caractérisé en ce que** la première couche est déposée au moyen d'une immersion du corps d'implant dans une solution qui contient de préférence du palladium et un électrolyte, par exemple, un électrolyte aqueux contenant un tensioactif ou contenant un hydrocarbure.

10. Procédé selon la revendication 9, **caractérisé en ce que** le corps de l'implant séjourne dans la solution pendant une durée d'environ 10 secondes à environ 30 secondes.

11. Procédé selon la revendication 8, **caractérisé en ce que** la première couche est déposée au moyen d'un traitement galvanique dans une solution aqueuse, où la solution aqueuse contient de préférence un sel conducteur soluble dans l'eau, par exemple, du dihydrogéno-phosphate de potassium, un agent de complexation, par exemple, de l'acide citrique, de l'acide éthylène diamine tétra acétique et/ou de l'acide tartrique droit, ainsi qu'un composé métallique contenant du palladium, par exemple, un composé faisant partie du groupe contenant un acétate trimère de palladium (II) et une tris (dibenzozylidène acétone) dipalladium.

12. Procédé selon l'une des revendications 8 à 11, **caractérisé en ce qu**'avant le dépôt de la première couche, au moins une partie de la surface corporelle est soumise à un traitement chimique par plasma dans une solution aqueuse pour la création d'une couche intermédiaire, chez laquelle une tension électrique générant le plasma est appliquée sur le corps de l'implant.

13. Procédé selon la revendication 12, **caractérisé en ce que** la solution aqueuse pour la génération de la couche intermédiaire présente un ou plusieurs ions choisis dans le groupe des ions phosphates, des ions potassium et des ions calcium.

14. Procédé selon l'une des revendications 8 à 13, **caractérisé en ce qu**'une deuxième couche, qui contient de préférence du parylène et/ou du stéarate de magnésium est déposée sur la première couche.
